# EUROPEAN PATENT APPLICATION

(11) **EP 4 338 727 A1**
(43) Date of publication of application: **20.03.2024**
(21) Application number: 22306347.0
(22) Date of filing: 14.09.2022
(51) Int. Cl.: A61K 9/00, A61K 39/235, A61K 47/12, A61K 47/40

(54) **ADENOVIRUS FORMULATIONS**

(71) Applicant: ROQUETTE FRERES, 62136 Lestrem (FR)
(72) Inventor: PENG, Tao, Singapore 138667 (SG); GOH, Yuan Hao, Lucas, Singapore 138667 (SG)
(74) Representative: Plasseraud IP

(57) **Abstract**

The invention relates to an improved adenovirus formulation comprising: a) a recombinant adenovirus; b) a citrate buffer; c) a cyclodextrin compound; and d) a salt, said formulation having a pH ranging from about 5.5 to about 6.5, and wherein said formulation is free of non-ionic detergent. The invention also relates to a method of preserving an adenovirus which comprises preparing said formulation.

## Description

### Technical Field

The invention pertains to the field of adenovirus formulations.

### Background Art

Patent EP3046536 B1 (JANSSEN) discloses a stable liquid adenovirus formulation comprising: a) a recombinant adenovirus; b) a citrate buffer; c) hydroxypropyl-beta-cyclodextrin (HBCD); d) a salt; e) a non-ionic detergent, wherein said formulation has a pH ranging between 5.5 and 6.5.

This successful technology, however, still needs to be improved, in particular, in relation to the adenoviral stability when stored.

### Technical Problem

It was thus an object of the invention to provide improved adenovirus formulations.

It was another object of the invention to provide an adenovirus formulation with good and/or improved stability, for example, formulations which are able to stabilize the adenovirus for longer periods of time, at storage temperatures as high as 40°C.

It was another object of the invention to solve the abovementioned problems by providing formulations having, moreover, the other properties required for these types of products, notably in terms of safety.

### Presentation of the invention

The inventors succeeded in improving the formulation of EP3046536 B1 by removing the non-ionic detergent from the adenovirus formulation.

As evidenced in the Example below, the cyclodextrin compound HBCD is sufficient to stabilize the formulation. Removing polysorbate 80 from such formulation does not negatively impact the stability of the adenovirus formulation.

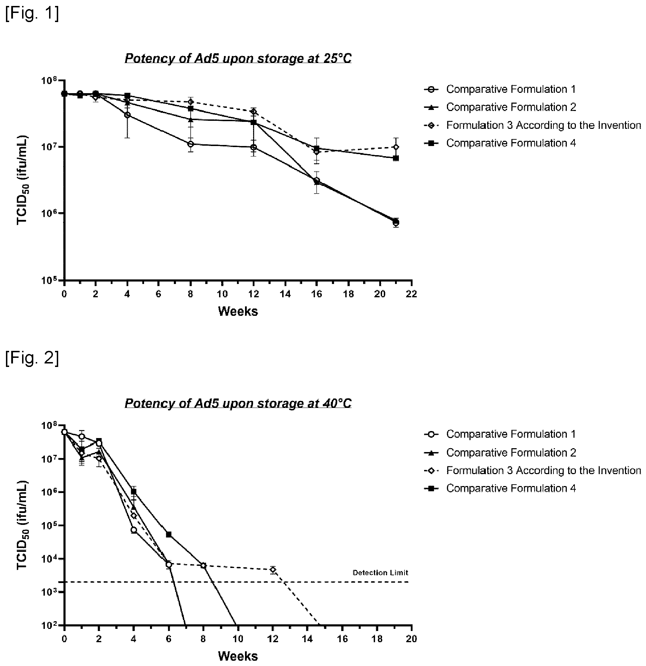

On the contrary and surprisingly, the removal of the non-ionic detergent improves the stability of the formulation, which completely goes against the teaching of EP3046536 B1, in which the non-ionic detergent was essential. This is apparent from Figure 2, showing that when using the non-ionic detergent-free formulation according to the disclosure the virus still shows potency 12 weeks after storage at high temperature (e.g., 40°C). As a comparison, the best formulation of EP3046536 B1 shows no potency 12 weeks after storage at high temperatures (e.g., 40°C).

### Brief Description of the invention

The invention first relates to an adenovirus formulation comprising: a) a recombinant adenovirus; b) a citrate buffer; c) a cyclodextrin compound; and d) a salt, said formulation having a pH ranging from about 5.5 to about 6.5, wherein said formulation is free of non-ionic detergent.

Preferably, said cyclodextrin compound is hydroxypropyl-beta-cyclodextrin (HPBCD).

Preferably, the citrate concentration of said formulation is ranging between about 5 mM and 30 mM.

Preferably, the concentration of cyclodextrin compound of said formulation is ranging between about 1% (w/w) to 10% (w/w).

Preferably, said salt is sodium chloride.

Preferably, the sodium chloride concentration of said formulation is ranging between about 20 mM to about 200 mM.

Preferably said salt is sodium chloride and said formulation is free of MgCl₂.

Preferably said formulation comprises: a) a recombinant adenovirus; b) a citrate buffer; c) a cyclodextrin compound; and d) sodium chloride; said formulation having a pH ranging from about 5.5 to about 6.5, and wherein said formulation is free of non-ionic detergent and free of MgCl₂.

Preferably, said formulation has a pH ranging between about 5.7 and 6.3, and comprises citrate at a concentration ranging between about 5 and 30 mM; a cyclodextrin compound at a concentration ranging between 1% (w/w) and 10% (w/w); NaCl at a concentration ranging between 20 mM and 200 mM.

Preferably, said formulation has a pH of about 6, and comprises citrate at a concentration of about 20 mM; cyclodextrin a compound at a concentration of about 5% (w/w); NaCl at a concentration of about 75 mM.

Preferably, said formulation further comprises a 2 or 4-carbon alcohol. Preferably, said 2 or 4-carbon alcohol is ethanol.

Preferably, the ethanol concentration of said formulation is ranging between about 0.1% (w/w) to 1% (w/w).

Preferably, said formulation has a pH ranging between about 5.7 and 6.3, and comprises citrate at a concentration ranging between about 5 and 30 mM; cyclodextrin compound at a concentration ranging between 1% (w/w) and 10% (w/w); NaCl at a concentration ranging between 20 mM and 200 mM; and ethanol at a concentration ranging between about 0.2% (w/w) and 0.6% (w/w).

Preferably, said formulation has a pH of about 6, and comprises citrate at a concentration of about 20 mM; cyclodextrin compound at a concentration of about 5% (w/w); NaCl at a concentration of about 75 mM, and ethanol at a concentration of about 0.4% (w/w).

Preferably, said formulation is a liquid formulation.

Preferably, said formulation is suitable for parenteral use.

The invention also relates to a method of preserving an adenovirus which comprises preparing said formulation.

### Brief Description of Drawings

Other features, details, and advantages will be shown in the following detailed description and on the figures, on which:
**Fig. 1**
   [Fig. 1] is a graph showing the potency of Ad5 upon storage at 25°C, in a formulation according to the invention or in comparative formulations.
**Fig. 2**
   [Fig. 2] is a graph showing the potency of Ad5 upon storage at 40°C, in a formulation according to the invention or in comparative formulations.

### Description of Embodiments

Figures and the following detailed description contain, essentially, some exact elements. They can be used to enhance understanding the invention and, also, to define the invention if necessary.

The invention first relates to an adenovirus formulation comprising: a) a recombinant adenovirus; b) a citrate buffer; c) a cyclodextrin compound; and d) a salt, said formulation having a pH ranging from about 5.5 to about 6.5, wherein said formulation is free of non-ionic detergent.

The formulations according to the disclosure comprise at least one recombinant adenovirus. The construction of adenoviral vectors is well understood in the art and involves the use of standard molecular biological techniques, such as those described in, for example, Sambrook et al., Molecular Cloning, a Laboratory Manual, 2d ed., Cold Spring Harbor Press, Cold Spring Harbor, N.Y. (1989), Watson et al., Recombinant DNA, 2d ed., Scientific American Books (1992), and Ausubel et al., Current Protocols in Molecular Biology, Wiley Interscience Publishers, NY (1995), and other references mentioned herein. In short, the adenoviral vector can be deficient in at least one essential gene function of the E1 region, e.g., the E1a region and/or the E1b region, of the adenoviral genome that is required for viral replication. As known to the skilled person for producing adenovirus, in case of deletions of essential regions from the adenovirus genome, the functions encoded by these regions have to be provided in trans, preferably by the producer cell, for instance integrated in the genome, or in the form of so-called helper adenovirus or helper plasmids, when producing the recombinant adenovirus.

Propagation of a recombinant adenovirus has been described in detail in: US patent 6,492,169 or in WO 03/104467 U.S. Pat. Nos. 5,559,099, 5,837,511, 5,846,782, 5,851,806, 5,994,106, 5,994,128, 5,965,541, 5,981,225, 6,040,174, 6,020,191, and 6,113,913, and Thomas Shenk, "Adenoviridae and their Replication", M. S. Horwitz, "Adenoviruses", Chapters 67 and 68, respectively, in Virology, B. N. Fields et al., eds., 3d ed., Raven Press, Ltd., New York (1996), which is incorporated here by reference. The replication-deficient adenoviral vector can be generated by using any species, strain, subtype, or mixture of species, strains, or subtypes, of an adenovirus or a chimeric adenovirus as the source of vector DNA (see for instance WO 96/26281, WO 00/03029). In certain embodiments of the present disclosure, serotypes of human adenovirus include any one of serotypes 2, 4, 5, 7, 11, 26, 34, 35, 36, 48, 49 or 50 or any hybrid or mutated adenovirus serotypes. In a preferred embodiment of the present disclosure the recombinant adenovirus is from human adenovirus serotype 5, 26 or 35. It is still preferably from human adenovirus serotype 5 (Adv5).

In further embodiments, the adenovirus of the disclosure is a simian adenovirus, preferably a chimpanzee or gorilla adenovirus. These adenoviruses generally have a low seroprevalence and/or low pre-existing neutralizing antibody titers in the human population.

In further embodiments, the adenovirus according to the disclosure further comprises heterologous nucleic acid. Suitable heterologous nucleic acid is well known to the skilled person, and for instance may include transgene open reading frames, for instance open reading frames coding for polypeptides against which an immune response is desired when the vector is used for vaccination purposes, e.g., transgenes suitable to generate an immune response against malaria (see e.g., WO 2004/055187), HIV, tuberculosis (see e.g., WO 2006/053871), certain viruses, etc, all well known to the skilled person. In fact, the nature of the transgene is not critical to the current disclosure, it may be any heterologous nucleic acid sequence, and hence needs no further elaboration here.

The term "stability" typically refers to the relative resistance to degradation of adenovirus particles in a formulation retaining its potency on the timescale of its expected usefulness.

Preferably, the formulation according to the disclosure shows higher potency, in particular higher infectious titer (infectious units/mL), as compared to a control formulation having a pH of 6 and composed of 20 mM citrate, 75mM NaCl, and 0.4% (w/w) ethanol after storage. Preferably, the formulation according to the disclosure shows higher potency, in particular, higher infectious titer (infectious units/mL), as compared to said control formulation, 8 or 12 weeks after storage at 40°C. Preferably, the formulation according to the disclosure shows higher potency, in particular higher infectious titer (infectious units/mL), as compared to said control formulation, 4 or 8 or 12 or 16 or 21 weeks after storage at 25°C.

The term "potency" as used herein refers to a measure of adenovirus activity expressed in terms of infectious units measured in a cell-based potency assay, preferably as described hereunder.

Preferably, the formulation according to the disclosure shows improved adenoviral-stability, in particular higher potency than, for instance, the best performing formulations known in the art (disclosed in Evans et al. 2004 and in EP3046536 B1), when stored in about the 2-8°C range while also being compatible with parenteral administration. These formulations can however also be stored at lower temperatures, e.g., -20°C or lower, -40°C or lower, -65°C or lower, -80°C or lower.

Preferably, the potency shows a decrease of not more than 0.3log per two years at 2-8°C.

Preferably, the composition according to the disclosure shows a decrease in potency of not more than 0.4log per 60 days and a decrease in titer of not more than 0.3log per 60 days in an accelerated stability study which study is performed by incubation of the formulations at 25°C ± 2°C during 1 to 3 months.

Preferably, the composition according to the disclosure also shows a decrease in potency of not more than 0.2log per 10 cycles in a study wherein vials are subjected to repeated freeze/thawing cycles followed by 24 hours of agitation at room temperature in a horizontal orientation at 200 rpm.

The expression "pharmaceutically acceptable excipient" classically refers to any inert substance that is combined with an active molecule such as a virus for preparing an agreeable or convenient dosage form. The "pharmaceutically acceptable excipient" is an excipient that is non-toxic to recipients at the dosages and concentrations employed, and is compatible with other ingredients of the formulation comprising the viral preparation. Examples of excipients are cryoprotectants, buffers, salts and inhibitors of free radical oxidation.

The expression "by-product" classically refers to undesired products, which detract or diminish the proportion of therapeutic/prophylactic adenovirus in a given formulation. Typical by-products include aggregates of the adenovirus and fragments of the adenovirus, resulting from e.g., protein denaturation, deamidation, hydrolysis or combinations thereof. Typically, aggregates are complexes that have a molecular weight greater than the isolated virus particle.

A formulation which improves the adenoviral stability, also named a "stable formulation" classically refers to a formulation in which the adenovirus therein essentially retains its physical and/or chemical integrity and/or biological activity upon storage, and/or to a formulation in which the adenovirus therein has higher physical and/or chemical integrity and/or biological activity upon storage as compared to a control formulation having a pH of 6 and composed of 20 mM citrate, 75mM NaCl, and 0.4% (w/w) ethanol.

Stability can be assessed by determining different characteristics such as the quantity (of adenovirus in a formulation), the potency, and/or other quality aspects of the adenovirus in the formulation over a period of time and under certain storage conditions. These characteristics of an adenovirus formulation can be measured at elevated temperatures (predictive for real-time temperatures) or under other stress conditions, for instance formulations can be subjected to incubation at 25°C or subjected to freeze/thaw cycles and agitation in order to study effects of different formulations maximizing shelf-life. Said characteristics which determine the stability may be determined by at least one of the methods selected from the group consisting of visual inspection, virus particle quantitative polymerase chain reaction, (vp-QPCR), QPCR-based Potency Assay (QPA), Virus Titration by TCID₅₀ (Tissue Culture Infectious Dose 50%) assay, Reverse Phase High Performance Liquid Chromotography (RP-HPLC) and Differential Centrifugal Sedimentation (DCS), Thermal Melting Assay (TMA), Turbidimetry, and Intrinsic Fluorescence.

### Virus particle quantitative polymerase chain reaction (vp-QPCR)

The vp-QPCR was developed for the quantification of adenovirus particles using primers that target a 100 bp region of the CMV promoter of the transgene cassette present within the adenovirus vector. Briefly, this QPCR method relies on the exonuclease activity of Taq polymerase, which results in degradation of a specific fluorescent probe annealed in the middle of the 100 bp amplicon. The probe is covalently linked to a light emitter and a quencher, and its degradation frees the emitter from the quencher with a consequent fluorescence emission proportional to the amount of template. Quantitative values are obtained from the threshold cycle (Ct), the cycle at which an increase in fluorescence signal exceeds a threshold value. The threshold for detection of DNA-based fluorescence is set slightly above background. The number of cycles at which the fluorescence exceeds the threshold is called the threshold cycle (Ct) or, according to the MIQE guidelines, quantification cycle (Cq) (Bustin et al, 2009). During the exponential amplification phase, the target DNA sequence doubles every cycle. For example, a DNA sample of which the Ct precedes that of another sample by 3 cycles contained 23 = 8 times more template. Consequently, a higher Ct value represents a lower amount of target DNA and a lower Ct value represents a high availability of target DNA. Absolute quantification can be performed by comparing a standard curve generated by a serial dilution of a stock adenovirus of which the concentration has been determined by the optical density at 260 nm (OD₂₆₀). The Ct values of the test material is plotted against the Ct values of the standard curve, which generates an accurate and precise number of vector particles.

When used as readout after incubation on E1 competent cells (QPA, see below), more degraded samples will lead to higher delta (t=0 subtracted) Ct values and more stabilizing formulations will lead to lower Ct values.

### Virus Titration by TCID₅₀ (Tissue Culture Infectious Dose 50%) assay

To quantify adenovirus potency, the TCID₅₀ assay is utilized to define the dilution of virus required to infect 50% of the cell monolayers, which results in the determination of the infectious titer of adenovirus calculated by using the Reed-Muench, Probit or Spearman-Karber analytical formula. The assay is based on the experimental observation of cytopathic effect (CPE) caused by infecting a carefully matched cell line (i.e., HEK293) with adenovirus. Dilutions of samples are inoculated onto HEK293 cell monolayers in a 96-well plate. The infection is allowed to proceed for 2-4 hours at 37°C. Wells were aspirated and replenished with medium that does not contain adenoviruses. Plates are incubated for an additional 3-7 days prior to detection of fluorescence exhibited by GFP encoded within the adenovirus DNA sequences. The TCID₅₀ value is calculated using either of the formulas mentioned above. A QPCR is performed on diluted cell lysates according to the method described above. When comparing relative differences in potency between formulations, this is a simple and reliable method.

### QPCR-based Potency Assay (QPA)

To quantify adenovirus potency, the QPA combines QPCR with a tissue culture-based infectivity assay. The assay is based on the experimental observation that the appearance of newly synthesized viral DNA is very rapid after inoculation of a cell-monolayer, and is proportional to the virus input concentration over a large range of multiplicity of infection (MOI). Dilutions of samples (non-endpoint diluted) are inoculated onto HEK293 cell monolayers in a 96-well plate. The infection is allowed to proceed for 3 hours at 35°C. Wells are aspirated and replenished with medium that does not contain adenoviruses. Plates are incubated for an additional 42 hours prior to cell lysis by means of Triton X-100 solution and a single freeze/ thaw step in order to release adenovirus DNA. A QPCR is performed on diluted cell lysates according to the method described above. The infectivity titer is calculated by comparison to a standard curve generated by the Ct values of a sample of known infectivity, which is determined by endpoint titration. Alternatively, the delta potency can be expressed directly as Ct values since the infectivity titer, or potency, is directly correlated to the Ct values. Especially in comparing relative differences in potency between formulations, this is a quick and reliable method.

### Reverse Phase High Performance Liquid Chromatography (RP-HPLC)

In order to determine some quality aspects of an adenovirus, one can analyze adenoviral protein profiles by Reverse Phase High Performance Liquid Chromatography (RP-HPLC). HPLC separates components of a mixture by using a variety of chemical interactions between the sample, the mobile phase (a buffer or solvent) and the stationary phase (a chromatographic packing material in a column). A high-pressure pump moves the mobile phase through the column and a detector shows the retention times (t_{R}; time between sample injection and the appearance of the peak maximum) of the molecules using UV absorbance detection at 280 nm. The separation of RP-HPLC is based on differences in hydrophobicity. The nonpolar stationary phase is made up of hydrophobic alkyl chains (chain lengths: C4, C8 and C18). The polar mobile phase is water with 0.1% trifluoroacetic (TFA). Compounds that bind to the columns are eluted using an increasing concentration of acetonitrile with 0.1% TFA. In general, an analyte with a larger hydrophobic surface area has a longer retention time, whereas the presence of polar groups reduce retention time. A typical adenoviral RP-HPLC profile consists of 10 or 14 proteins, including core protein (VII), penton base (III) and hexon (II).

### Differential Centrifugal Sedimentation (DCS)

DCS is a method to measure particle size distributions (aggregation) by sedimentation. In a disc centrifuge, particles settle in a sucrose gradient (of known viscosity and density) under high gravitational forces according to Stokes' law. Sedimentation velocity increases with the square of the particle diameter, so particles that differ in size by only a few percent settle at significantly different rates. The time needed to reach the detector is used to calculate the size of the particles. The measurement range for this method is about 0.02 to 30 microns.

### Thermal melting assay (TMA)

The thermal melting assay (TMA) can be used to determine the melting temperature (Tₘ) of adenovirus in experimental formulations, which is the temperature where the viral capsid denatures. This viral disintegration can be measured real-time using a dsDNA intercalating fluorescent dye. This fluorescent dye only gives a fluorescence signal when bound to DNA, which is released when the viral particle disintegrates. The exponential fluorescence increase upon capsid melting can be measured using a common QPCR machine during a stepwise increase in temperature. Samples are diluted to the same concentration (range is 4×10⁹ to 1×10¹² vp/mL) in the specific formulations and mixed with SYBRGreen dye (IX final concentration) in a volume of 50 µL. The temperature was increased 0.5°C per 30 seconds starting from 30°C up to 79°C. From the fluorescent raw data first and second derivatives are calculated and melting temperature is read at the intercept of the second derivative with the x-axis. Higher melting temperatures (Tₘ) may be indicative of a more stabilizing formulation.

### Turbidity assay

Turbidimetry measures the loss of intensity of transmitted light due to scattering of particles in samples (apparent absorbance), detected at a wavelength where the molecules in the sample do not absorb light (e.g., 350 nm for samples in which proteins are the main chromophore). When molecules aggregate or form supramolecular complexes, the light scattering, which was random when coming from the separate particles, now becomes coherent, and thereby the measured intensity increases. This makes light scattering and turbidimetry useful techniques for detecting aggregation and complex formation or dissociation.

In the turbidity assay, samples are transferred in triplicate to a UV-transparent, flat-bottom microplate. The plate is covered with a UV-transparent seal. Absorbance spectra are recorded by a microplate reader between 230 and 500 nm, and the absorbance at 975 nm is measured to determine and possibly correct for differences in optical pathlength. Control samples consisting of the formulations without the adenovirus are included in the assay to correct for scattering or absorbing matrix components if required. The apparent absorbance at 350 nm is used as a quantitative measure for turbidity.

The turbidity assay is stability-indicating for adenovirus samples. Virus aggregation leads to an increase in turbidity and capsid dissociation to a decrease. The assay precision is < 5% (CV%) at turbidity values > 1 NTU.

The obtained turbidity for stressed samples should always be compared to the control samples. Since an increase or decrease after applied stress is dependent on the degradation pathway and specific for each Active Pharmaceutical Ingredient (API), it cannot be predicted. A change (higher or lower) compared to the t=0 samples is indicative of a less stable formulation. Stressed samples comparable to the t=0 samples are expected to be more stable.

### Intrinsic fluorescence assay

The adenoviral capsid proteins contain aromatic amino acids that reemit light after excitation, in particular tryptophan and to a lesser extent tyrosine and phenylalanine. The emission maximum and quantum yield of tryptophan depend strongly on the polarity of its environment. In a polar, aqueous environment (e.g., the surface of a globular protein) the quantum yield is relatively low, while in an apolar environment (e.g., the inside of an aggregate) the quantum yield increases. This feature makes tryptophan fluorescence a useful tool for studying protein conformational change, aggregation, and molecular interactions.

In the intrinsic fluorescence assay, samples are transferred in triplicate to a UV-transparent, flat-bottom microplate. The plate is covered with a UV-transparent seal. Tryptophan fluorescence is measured by a microplate reader using an excitation filter with a center wavelength of 280 nm and a bandwidth of 10 nm, and an emission filter with a center wavelength of 340 nm and a bandwidth of 10 nm. Bottom optic is used to minimize the influence of the seal and the meniscus shape.

The fluorescence intensity is known in the art to be a sensitive measure of adenovirus stability. Either an increase or a decrease may be observed upon stress, depending on the nature of the changes occurring in the sample. Protein unfolding and capsid dissociation is expected to lead to a decrease in intrinsic fluorescence, and aggregation is expected to lead to an increase. The precision of the assay is < 5% (CV%) in the range used.

The obtained fluorescence for stressed samples should always be compared to the control samples. Since an increase or decrease after applied stress is dependent on the degradation pathway and specific for each Active Pharmaceutical Ingredient (API), it cannot be predicted. A change (higher or lower) compared to the t=0 samples is indicative of a less stable formulation. Stressed samples remaining close to the t=0 sample values are more stable.

An adenovirus "retains its physical stability" in a pharmaceutical formulation, for example if it, amongst others, shows minimal loss (i.e., 0.3log/2 years) in terms of quantity and potency, and displays no major protein modifications. Additionally, no signs of aggregation, precipitation, change of colour and/or clarity upon visual examination should be observed.

"About" as used in the present application means ±10%, unless stated otherwise.

These formulations, which are able to stabilize an adenovirus, comprise a citrate buffer, a cyclodextrin compound, and a salt, as well as optional additional components which enhance stability to the added virus. The pH of said citrate buffer is ranging from about 5.5 to about 6.5. The formulations according to the disclosure are free of non-ionic detergent, in particular free of polyoxyethylene sorbitan fatty acid esters, e.g., Polysorbate-80 (Tween 80^{®}), Polysorbate-60 (Tween 60^{®}), Polysorbate-40 (Tween 40^{®}), Polysorbate-20 (Tween 20^{®}), and of the Pluronic^{®} series of non-ionic surfactants (e.g., Pluronic^{®} 121). Still preferably, the formulations according to the disclosure are free of any kind of detergent.

The expression "free of" in connection with a particular ingredient or compound typically indicates that the formulations according to the present disclosure contain a concentration of said particular ingredient or compound lower than 0.001 % (w/w), preferably lower than 0.0001 %, even more preferably equal to 0.0000% (w/w). Still preferably, said compound or ingredient is completely absent of the formulation.

Alternatively or complementarily, the formulations according to the present disclosure comprise: a) a recombinant adenovirus; b) a citrate buffer; c) a cyclodextrin compound; and d) a salt, said formulation having a pH ranging from about 5.5 to about 6.5, and having a concentration of non-ionic detergents or of any kind of detergents lower than 0.001 % (w/w), preferably lower than 0.0001 %, even more preferably equal to or lower than 0.0000% (w/w). Still preferably, said detergents are completely absent of the formulation.

The formulations of the present disclosure provide stability to adenoviruses at varying virus concentrations, mono- or multivalent, and may be administered to a variety of vertebrate organisms, preferably mammals and especially humans. The stabilized viral formulations of the present disclosure are adenoviral-based compositions, which can, for instance, be administered as a vaccine that may offer a prophylactic advantage to previously uninfected individuals and/or provide a therapeutic effect.

A preferred aspect of the disclosure is an adenovirus formulation, comprising recombinant adenoviruses (i.e., an adenovirus containing a whole or a portion of a transgene which is expressed within the target host subsequent to host administration, such as in any mammalian/human gene therapy- or gene vaccination-based methodology available to the skilled artisan) which shows enhanced stability characteristics described herein with a virus concentration in the range from about 1×10⁷ vp/mL (virus particles/mL) to about 1×10¹³ vp/mL. A more preferred range is from about 1×10⁹ to 1×10¹³ vp/mL, with an especially preferred virus concentration being from about 1×10¹⁰ to 5×10¹² vp/mL. Therapeutic or prophylactic compositions of the formulations of the present disclosure can be administered to an individual in amounts sufficient to treat or prevent the respective disorder. The effective amount for human administration may, of course, vary according to a variety of factors such as the individual's condition, weight, sex and age. Other factors include the mode of administration. In a preferred embodiment, the formulations of the present disclosure are suitable for parenteral use.

The formulations of the present disclosure are citrate buffered solutions having a pH of 5.5 to 6.5, further comprising a cyclodextrin compound, preferably hydroxypropyl-beta-cyclodextrin (HPBCD), and optionally comprising a two or four carbon alcohol. Unexpectedly, said combination has proven to be an outstanding formulation for preserving of quantity, potency (infectivity) and quality of adenoviruses, as demonstrated herein.

In a preferred embodiment, the concentration of citrate buffer, is ranging between about 5 mM and 30 mM, e.g., between about 5 mM and 25 mM, e.g., between about 10 mM and 25 mM, e.g., about 20 mM.

Another essential component in these formulations is the cyclodextrin compound.

The expression « cyclodextrin compound» classically refers to a mixture of cyclodextrin molecules, further comprising the substances resulting from its manufacturing process. Such cyclodextrin compound might be a native or a substituted cyclodextrin. Contrary to chemical substances with well-defined structure, substituted cyclodextrins generally are a mixture of substituted cyclodextrin molecules having different substitution profiles, and which are thus structurally different.

In a preferred embodiment, the cyclodextrin compound according to the disclosure is substituted. It can or instance be selected from hydroxypropyl-beta-cyclodextrin, methyl-beta-cyclodextrin, sulfobutylether-beta-cyclodextrin, or from a mixture thereof. It is preferably substituted with alkyl group and/or hydroxyalkyl group, preferably having 1 to 5 carbon atoms, preferably 1 to 4, preferably 1 to 3, preferably 1 or 3. It is preferably selected from hydroxypropyl-cyclodextrin, methyl-cyclodextrin, or from a mixture thereof. It is preferably hydroxypropyl-cyclodextrin, in particular hydroxypropyl-β-cyclodextrin (HPBCD).

Preferably, the substituted cyclodextrin according to the disclosure, preferably HPBCD, has an average molar substitution degree (MS) equal to or higher than 0.10, preferably equal to or higher than 0.20, preferably equal to or higher than 0.30, preferably equal to or higher than 0.40, preferably equal to or higher than 0.50. Preferably, this MS is equal to or lower than 1.50, preferably equal to or lower than 1.40, preferably equal to or lower than 1.30, preferably equal to or lower than 1.20, preferably equal to or lower than 1.10, preferably equal to or lower than 1.00.

It is reminded that the "average molar substitution degree (MS)" refers to the average number of added substituents per anhydroglucose unit. It should be noted that this MS is different from the average molecular substitution degree (DS), which refers to the average number of added substituents per cyclodextrin molecule, and which is, as a consequence, function of the number of anhydroglucose units forming the original cyclodextrin. For instance, for alkylated beta-cyclodextrins, this DS is equal to 7 times the MS, as the β-cydodextrins are made of 7 anhydroglucose units.

In a preferred embodiment, the hydroxypropyl-cyclodextrin according to the disclosure (e.g., HPBCD) has:
(i) a MS selected from 0.50 to 0.75, preferably from 0.55 to 0.70, preferably from 0.58 to 0.68; and/or,
(ii) the following substitution profile, as determined by electrospray ionization - Mass spectrometry (ESI-MS):
   - HP0 : equal to or lower than 5 %, preferably equal to or lower than 4 %, preferably equal to or lower than 3 %, preferably equal to or lower than 2 %, preferably equal to or lower than 1 %, preferably equal to 0 %; and/or,
   - HP1: equal to or lower than 5 %, preferably equal to or lower than 4 %, preferably equal to or lower than 3 %, preferably equal to or lower than 2 %, preferably equal to or lower than 1 %; and/or,
   - HP2: equal to or lower than 5 %, preferably equal to or lower than 4 %, preferably equal to or lower than 3 %, preferably equal to or lower than 2 %; and/or,
   - HP3: selected within the range of from 0 to 15 %, preferably of from 1 to 10 %, preferably of from 1 to 8 %, preferably of from 2 to 6 %; and/or,
   - HP4: selected within the range of from 0 to 25 %, preferably of from 2 to 20 %, preferably of from 4 to 15 %, preferably of from 7 to 13 %; and/or,
   - HP5: selected within the range of from 1 to 40 %, preferably of from 5 to 35 %, preferably of from 10 to 30 %, preferably of from 15 to 25 %; and/or,
   - HP6: selected within the range of from 5 to 50 %, preferably of from 10 to 40 %, preferably from 15 to 35 %, preferably of from 20 to 30 %; and/or,
   - HP7: selected within the range of from 5 to 50 %, 10 to 40 %, preferably of from 15 to 35 %, preferably of from 20 to 30 %; and/or,
   - HP8: selected within the range of from 2 to 30 %, preferably of from 2 to 25 %, preferably of from 5 to 20 %, preferably of from 8 to 15 %; and/or,
   - HP9: selected within the range of from 1 to 10 %, preferably of from 1 to 8 %, preferably of from 2 to 5 %; and/or,
   - HP10: equal to or lower than 5 %, preferably equal to or lower than 4 %, preferably equal to or lower than 3 %, preferably equal to or lower than 2 %, preferably equal to or lower than 1 %; and/or,
   - HP≥11: equal to or lower than 5 %, preferably equal to or lower than 4 %, preferably equal to or lower than 3 %, preferably equal to or lower than 2 %, preferably equal to or lower than 1 %, preferably equal to 0 %;
   these percentages being expressed with respect to the sum of the signals obtained for each substitution for which the signal was higher than the one of the background.

It is understood that "signal" refers to the area under the curve of the ion(s) corresponding to the substitutions degree(s) of interest.

In the present disclosure, the substitution profile is determined by ESI-MS, preferably by calculating the average of the measurements performed in triplicate. For determining this substitution profile, it is possible to proceed according to the following method:
- A solution hydroxypropyl-cyclodextrin is prepared at 1 g.L⁻¹ (w/v) in methanol: water (50/50, v/v) with 1 mM sodium acetate. Infusion is performed for 1 min at 10 µL/min and data are recorded. In between two following injections, 500 µL of methanol: water (50/50, v/v) are injected to wash the ion source.
- ESI parameters: Spray voltage: 5 kV; Sheath gas: 9; Auxiliary gas: 2; Sweep gas: 0; Spray voltage: 5 kV; Capillary voltage: 23 V; Capillary temperature: 275°C; Tube lens: 80 V
- MS parameters: Full scan; Scan ranges: 50-2000 m/z; Mass range: normal; Scan rate: enhanced; Data acquisition time: 1 min.
- For each substituted hydroxypropyl-cyclodextrin species (named as HPX, X being the number of hydroxypropyl substitutions), the corresponding ion current (XIC) is integrated and compared to the sum of all HPX ion currents. As the sodium adduct is the most intense hydroxypropyl-cyclodextrin ion (almost 100 times superior to [M+H]⁺ or [M+NH4]⁺), its peak area is integrated to estimate the proportion of the corresponding HPX molecule.

Such hydroxypropyl-cyclodextrin is commercially available. For example, mention can be made of KLEPTOSE^{®} HPB (ROQUETTE FRERES).

In a preferred embodiment, the concentration of cyclodextrin compound (e.g., HPBCD) is ranging between about 1% (w/w) to 10% (w/w), e.g., between about 3% (w/w) to 8% (w/w), e.g., between about 4% (w/w) to 6% (w/w), e.g., about 5% (w/w).

An additional component of the formulations of the present disclosure is salt. Salt enhances viral stability. A purpose of inclusion of a salt in the formulation is to attain the desired ionic strength or osmolality and additionally optimize electrostatic interactions. Salt is present at an osmolality which is physiologically acceptable to the host. Contributions to ionic strength may come from ions produced by the buffering compound as well as from the ions of non-buffering salts. Salts that are appropriate for the formulations of the present disclosure include but are not limited to sodium chloride (NaCI), Calcium chloride (CaCl₂) or manganese chloride (MnCl₂). In contrast to the prior art, magnesium chloride (MgCl₂) was shown to be detrimental to adenoviral-stability. Therefore, in a preferred embodiment the formulation according to the present disclosure is free of magnesium chloride (MgCl₂), or has a concentration of MgCl₂. lower than 0.001% (w/w), preferably lower than 0.0001% (w/w) and even more preferably equal to or lower than 0.0000% (w/w). Still preferably, the MgCl₂. is completely absent from the formulation.

In a preferred embodiment, the adenovirus formulation according to the present disclosure comprises sodium chloride (NaCI).

In a more preferred embodiment the adenovirus formulations according to the present disclosure comprise: a) a recombinant adenovirus; b) a citrate buffer; c) a cyclodextrin compound; and d) NaCl; said formulation having a pH ranging from about 5.5 to about 6.5, wherein said formulations are free of MgCl₂. and free of non-ionic detergent, in particular free of MgCl₂. and free of polyoxyethylene sorbitan fatty acid esters, e.g., Polysorbate-80 (Tween 80^{®}), Polysorbate-60 (Tween 60^{®}), Polysorbate-40 (Tween 40^{®}), Polysorbate-20 (Tween 20^{®}), and of the Pluronic^{®} series of non-ionic surfactants (e.g., Pluronic^{®} 121). Still preferably, the formulations according to the disclosure are free of MgCl₂. and free of any kind of detergent.

Also a preferred embodiment refers to the adenovirus formulation according to the present disclosure comprising: a) a recombinant adenovirus; b) a citrate buffer; c) a cyclodextrin compound; and d) NaCl; said formulation having a pH ranging from about 5.5 to about 6.5, having a concentration of non-ionic detergents or of any kind of detergents lower than 0.001 % (w/w), preferably lower than 0.0001 %, even more preferably equal to 0.0000% (w/w), and having a concentration of MgCl₂. lower than 0.001% (w/w), preferably lower than 0.0001% (w/w) and even more preferably equal to 0.0000% (w/w). still preferably said detergents and MgCl₂. are completely absent from the formulation.

In a preferred embodiment, the concentration of sodium chloride is ranging between about 10 mM and 250 mM, e.g., between about 20 mM and 200 mM, e.g., between about 30 mM and 150 mM, e.g., between about 50 mM and 100 mM, e.g., about 80 mM.

A particular embodiment according to the present disclosure refers to an adenovirus formulation consisting of a) a recombinant adenovirus; b) a citrate buffer; c) a cyclodextrin compound; d) NaCl, and e) EDTA and/or a 2 or 4-carbon alcohol, preferably ethanol, said formulation having a pH ranging from about 5.5 to about 6.5.

In an embodiment, the virus formulation according to the present disclosure further comprises EDTA. In that case, the concentration of EDTA is preferably ranging between about 0.05 mM to 0.2 mM, e.g., between about 0.05 mM to 0.15 mM, e.g., between about 0.08 mM to 0.12 mM, e.g., about 0.1 mM.

In a preferred embodiment, the virus formulation according to the present disclosure further comprises ethanol. In a more preferred embodiment, the concentration of ethanol is ranging between about 0.1% (w/w) to 1% (w/w), e.g., between about 0.2% (w/w) to 0.8% (w/w), e.g., between about 0.2% (w/w) to 0.6% (w/w), e.g., about 0.4% (w/w).

In a preferred embodiment, when the virus formulation according to the present disclosure comprises ethanol, it must not necessarily comprise EDTA at the same time.

In view of the discussion above, the present disclosure relates to a formulation containing an adenovirus, such as a recombinant Ad5, Ad26 or Ad35, that can e.g., be used in gene therapy and/or gene vaccination applications, which show improved stability properties as compared to the best performing formulation known in the art (disclosed in Evans et al. 2004 and in EP3046536 B1) and which at least contains a citrate buffer, a cyclodextrin compound, preferably HBCD, as a cryoprotectant, and a salt.

A preferred embodiment of the present disclosure relates to such a recombinant adenovirus formulation which is citrate buffered to a pH ranging from about 5.5 to about 6.5, and further comprising a cyclodextrin compound, preferably hydroxypropyl-beta-cyclodextrin (HBCD), a salt, and a 2 or 4-carbon alcohol.

In a preferred embodiment according to the present disclosure, the formulation comprises a citrate buffer with a pH ranging from about 5.5 to about 6.5, a cyclodextrin compound as the cryoprotectant (preferably HPBCD), NaCl as the salt, and a 2- or 4-carbon alcohol as an additional unprecedented cryoprotectant.

In another preferred embodiment according to the present disclosure, the formulation comprises a citrate buffer with a pH ranging from about 5.5 to about 6.5, a cyclodextrin compound as the cryoprotectant (preferably HPBCD), NaCl as the salt, and EDTA.

In another preferred embodiment according to the present disclosure, the formulation comprises a citrate buffer with a pH ranging from about 5.5 to about 6.5, a cyclodextrin compound as the cryoprotectant (preferably HPBCD), NaCl as the salt, and ethanol as an additional unprecedented cryoprotectant.

In another preferred embodiment according to the present disclosure, the formulation comprises a citrate buffer with a pH ranging from about 5.5 to about 6.5, a cyclodextrin compound as the cryoprotectant (preferably HPBCD), NaCl as the salt. This formulation further comprises ethanol and is free of EDTA.

In a preferred embodiment according to the present disclosure the formulation has a pH ranging between about 5.9 and 6.2, and comprises citrate at a concentration ranging between about 10 and 25 mM; a cyclodextrin compound (preferably HBCD) at a concentration ranging between 4% (w/w) and 6% (w/w); NaCl at a concentration ranging between 70 mM and 100 mM; and ethanol at a concentration ranging between about 0.3% (w/w) to 0.45% (w/w).

In another preferred embodiment according to the present disclosure the formulation has a pH ranging between about 5.8 and 6.2, and comprises citrate at a concentration ranging between about 15 and 25 mM; a cyclodextrin compound (preferably HBCD) at a concentration ranging between 3% (w/w) and 8% (w/w); NaCl at a concentration ranging between 50 mM and 100 mM; and EDTA at a concentration ranging between about 0.05 mM to 0.15 mM.

In another preferred embodiment according to the present disclosure the formulation has a pH ranging between about 5.9 and 6.2, and comprises citrate at a concentration ranging between about 10 and 25 mM; a cyclodextrin compound (preferably HBCD) at a concentration ranging between 4% (w/w) and 6% (w/w); NaCl at a concentration ranging between 70 mM and 100 mM; and EDTA at a concentration ranging between about 0.05 mM to 0.15 mM.

In an even more preferred embodiment of the present disclosure the formulation is buffered with about 20 mM citrate to a pH of about 6; the cyclodextrin compound (preferably HBCD) is present at a concentration of about 5% (w/w); NaCl is present at a concentration of about 75 mM; and EDTA is present at a concentration of about 0.1 mM.

In an even more preferred embodiment of the present disclosure the formulation is buffered with about 20 mM citrate to a pH of about 6; the cyclodextrin compound (preferably HBCD) is present at a concentration of about 5% (w/w); NaCl is present at a concentration of about 75 mM; and ethanol is present at a concentration of about 0.4% (w/w). Additionally, combinations of the above mentioned factors can be used.

In one embodiment the formulations according to the present disclosure are contained in a vial such as e.g., DIN 2R type I borosilicate glass vial. In another embodiment, the formulations are contained in a bag. Bags that contain the formulations of the present disclosure may comprise layers made of e.g., Ethylene Vinyl Acetate Copolymer (EVA) or Ethyl Vinyl Alcohol (EVOH). In yet another embodiment of the present disclosure, the formulations are contained in a syringe.

The recombinant adenovirus formulations described herein can be administered to the vertebrate host (preferably a mammalian host and especially a human recipient) by any means known in the art. These routes of delivery include but are not limited to intramuscular injection, intraperitoneal injection, intravenous injection, inhalation or intranasal delivery, oral delivery, sublingual administration, subcutaneous administration, transdermal administration, intradermal administration, intraductal salivary gland administration, transcutaneous administration or percutaneous administration. In a preferred embodiment, the formulation of the present disclosure is compatible with parenteral administration.

In accordance with the formulations disclosed herein, the present disclosure also relates to methods of preserving an adenovirus which comprise preparing virus containing formulations as disclosed herein, such formulations which result in improved viral stability 8 or 12 weeks after storage at 40°C.

In accordance with the formulations disclosed herein, the present disclosure also relates to methods of preserving an adenovirus which comprise preparing virus containing formulations as disclosed herein, such formulations which result in improved viral stability 4 or 8 or 12 or 16 or 21 weeks after storage at 25°C.

Preferably, the formulations have an improved stability when stored below -65°C and/or in about the 2-8 °C range while also being compatible with parenteral administration, especially parenteral administration to humans.

It is in particular the formulations have improved stability as compared to a control formulation having a pH of 6 and composed of 20 mM citrate, 75mM NaCl, and 0.4% (w/w) ethanol.

The following examples are provided to illustrate the present invention without, however, limiting the same hereto.

### Examples

### I. Material and methods

Ad-Cre-GFP (Signagen Laboratories, Cat. No. SL101086) was purchased for the formulation of study samples at a titre of 2.5E+9 vgs/mL. Virus vectors were diluted in four buffers having the formula shown in Table 1. Re-formulated samples were aliquoted into 100 µL glass vials prior to crimping and parafilming.

**[Table 1]**

| | Com parative Formulation 1 | Com parative Formulation 2 | Formulation 3 according to the invention | Com parative Formulation 4 |
|---|---|---|---|---|
| citrate | 20 mM | 20 mM | 20 mM | 20 mM |
| NaCl | 75 mM | 75 mM | 75 mM | 75 mM |
| Polysorbate 80 | / | 0.02 % | / | 0.02 % |
| HPBCD (KLEPTOSE^{®} HPB) | / | / | 5% | 5% |
| ethanol | 0.4 % | 0.4 % | 0.4 % | 0.4 % |
| pH | 6 | 6 | 6 | 6 |
| Adenovirus (Adv5) | 2.84E+08 vqs/uL | 2.84E+08 vqs/uL | 2.84E+08 vqs/uL | 2.84E+08 vqs/uL |

### II. Evaluation of the formulations

Virus vector samples were then subjected to temperature stress at 40°C and 25°C, with 75% and 60% relative humidity, respectively. Time points were collected in the mornings of the days, and samples were stored at -80°C until required for analysis.

HEK393 cells were seeded into 96-well plates at 4000 cells/well in 150µL of complete growth media (MEM + 10% FBS) for 4 days to reach ~70-80% confluency on day of infection. 20uL of each Adv5 stability samples was serially diluted with complete growth media (i.e., 50x, 500x, 5000x, 50000x, 500000x). Following the removal of used cell media, 50uL of each virus vector dilution were added to 10 wells of the 96-well plate (i.e., row-wise). Virus vector samples were incubated with the cells at 37°C with 5% CO₂ for 4 hours. Thereafter, 50uL of fresh complete growth media were added to all wells.

Three- or seven-days post-infection, plates were subjected to TECAN analysis at 485nm (excitation) and 535nm (emission). GFP absorbance values for each sample were obtained after deducting that of the negative cell-only wells.

The Reed and Muench method was used to determine the titre (i.e., TCID₅₀/mL) of each virus by counting the percentage of positive wells at each dilution (i.e., out of a row of 10 wells):

log10 50% end point dilution = log10 of dilution showing a mortality next above 50% - (difference of logarithms × logarithm of dilution factor)

Results are presented in Figures 1 and 2.

### III. Conclusion

The non-ionic detergent free formulation according to the invention shows very good stability. When stored at 25°C, the stability is as good as comparative formulation 4 according to patent EP3046536 B1. It seems to be even slightly better after 21 weeks.

When stored in more stressful conditions (at 40°C), the non-ionic detergent free formulation according to the invention also shows very good stability. The stability is even better than the stability of comparative formulation 4 according to patent EP3046536 B1: after 12 weeks of storage at 40°C, comparative formulation 4 shows no detectable potency, whereas formulation 3 according to the invention still shows detectable potency.

## Claims

1. An adenovirus formulation comprising: a) a recombinant adenovirus; b) a citrate buffer; c) a cyclodextrin compound; and d) a salt, said formulation having a pH ranging from about 5.5 to about 6.5, wherein said formulation is free of non-ionic detergent.

2. The formulation of claim 1, wherein said cyclodextrin compound is hydroxypropyl-beta-cyclodextrin (HPBCD).

3. The formulation according to any of claims 1 or 2, wherein the citrate concentration is ranging between about 5 mM and 30 mM.

4. The formulation according to any of claims 1 to 3, wherein the concentration of cyclodextrin compound is ranging between about 1% (w/w) to 10% (w/w).

5. The formulation according to any of claims 1 to 4, wherein the salt is sodium chloride (NaCI).

6. The formulation according to any of claims 1 to 5, wherein the sodium chloride (NaCl) concentration is ranging between about 20 mM to about 200 mM.

7. The formulation according to any of claims 1 to 6, wherein said formulation has a pH ranging between about 5.7 and 6.3, and comprises citrate at a concentration ranging between about 5 and 30 mM; a cyclodextrin compound at a concentration ranging between 1% (w/w) and 10% (w/w); NaCl at a concentration ranging between 20 mM and 200 mM.

8. The formulation according to any of claims 1 to 7, wherein said formulation has a pH of about 6, and comprises citrate at a concentration of about 20 mM; cyclodextrin a compound at a concentration of about 5% (w/w); NaCl at a concentration of about 75 mM.

9. The formulation according to any of claims 1 to 8, wherein the formulation further comprises a 2 or 4-carbon alcohol.

10. The formulation of claim 9, wherein the 2 or 4-carbon alcohol is ethanol.

11. The formulation of claim 10, wherein the ethanol concentration is ranging between about 0.1% (w/w) to 1% (w/w).

12. The formulation of any of claims 10 or 11, wherein said formulation has a pH ranging between about 5.7 and 6.3, and comprises citrate at a concentration ranging between about 5 and 30 mM; cyclodextrin compound at a concentration ranging between 1% (w/w) and 10% (w/w); NaCl at a concentration ranging between 20 mM and 200 mM; and ethanol at a concentration ranging between about 0.2% (w/w) and 0.6% (w/w).

13. The formulation of claim 12, wherein said formulation has a pH of about 6, and comprises citrate at a concentration of about 20 mM; cyclodextrin compound at a concentration of about 5% (w/w); NaCl at a concentration of about 75 mM, and ethanol at a concentration of about 0.4% (w/w).

14. The formulation according to any of claims 1 to 13, wherein said formulation is a liquid formulation.

15. The formulation according to any of claims 1 to 14, wherein said formulation is suitable for parenteral use.

16. A method of preserving an adenovirus which comprises preparing a formulation according to any one of claims 1-15.
